# EUROPEAN PATENT SPECIFICATION

(11) **EP 2 183 267 B1**
(45) Date of publication and mention of the grant of the patent: **20.08.2014**
(21) Application number: 08786343.7
(22) Date of filing: 23.07.2008
(51) Int. Cl.: C07J 31/00, C07J 1/00, A61K 31/565, A61P 35/00, A61P 5/32

(54) **PROCESS FOR PREPARING 7-ALPHA-[9-(4,4,5,5,5-PENTAFLUOROTHIOPENTYL) NONYL]ESTRA-1,3,5(10)-TRIENE-3,17-BETA-DIOL**
VERFAHREN ZUR HERSTELLUNG VON 7-ALPHA-[9-(4,4,5,5,5-PENTAFLUORTHIOPENTYL)NONYL]ESTRA-1,3,5(10)-TRIEN-3,17-BETA-DIOL
PROCÉDÉ DE PRÉPARATION DU 7 -ALPHA-[9-(4,4,5,5,5-PENTAFLUOROTHIOPENTYL)NONYL]ESTRA-1,3,5(10)-TRIÈNE-3,17-BETA -DIOL

(30) Priority: 23.07.2007 IT MI20071479
(43) Date of publication of application: 12.05.2010
(73) Proprietor: INDUSTRIALE CHIMICA S.r.l., 20145 Milano (IT)
(72) Inventor: PIROVANO, Silvio, I-21013 Gallarate (IT); MENNA, Lorenzo, I-20099 Sesto San Giovanni (IT); LENNA, Roberto, I-20010 San Giorgio Su Legnano (IT)
(74) Representative: Palladino, Saverio Massimo
(86) International application number: PCT/EP2008/059651
(87) International publication number: WO 2009/013310

(56) References cited:
- WO-A-2005/077968
- WO-A-2006/015081

## Description

### FIELD OF THE INVENTION

The present invention relates to the field of processes for steroid synthesis and in particular to a process for preparing 7α-[9-(4,4,5,5,5-pentafluorothiopentyl)nonyl]estra-1,3,5(10)-triene-3,17β-diol, a precursor of steroids with hormonal activity, and of Fulvestrant in particular.

### STATE OF THE ART

The steroid of the below indicated formula, whose chemical name is 7α-[9-(4,4,5,5,5-pentafluorothiopentyl)nonyl]estra-1,3,5(10)-triene-3,17β-diol 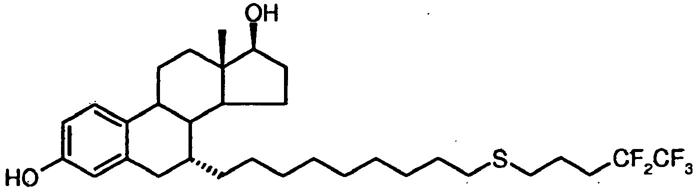 is a product known in the literature, and is described with its relative synthesis in patents EP 0138504 B1 and DE 4218743 A1 and in patent application WO 2005/077968.

The starting products for preparing 7α-[9-(4,4,5,5,5-pentafluorothiopentyl)nonyl]estra-1,3,5(10)-triene-3,17β-diol are, respectively, 17β-acetyloxyestra-4,6-dien-3-one for EP 0138504 and 17β-acetyloxy-3-methoxyestra-1,3,5(10)-trien-6-one for DE 4218743.

The critical point in both synthesis paths is the introduction of a suitable alkyl substituent in position 7 which, when further transformed, will give rise to the chain in the product of this invention.

In DE 4218743 the 17β-acetyloxy-3-methoxyestra-1,3,5(10)-trien-6-one must be transformed into the corresponding 17β-acetyloxy-3-methoxy-6-(phenylsulphonyl)estra-1,3,5(10),6-tetraene before being alkylated with 9-(t-butyldimethylsilyloxy)non-1-yne with an alkylation yield of 43% after column chromatography.

No information is provided on the α/β ratio of the mixture obtained.

In EP 0138504 the alkylation takes place by addition in position 7 of a Grignard reagent, obtained from 9-t-butyldimethylsilyloxy-nonyl bromide. In this case, information on the α/β ratio of the obtained mixture is supplied by WO 02/32922 A1 wherein, in the general description, the synthesis described in US 4,659,516, corresponding to EP 0138504, confirms an α/β ratio of 1.9:1.

To be noted is that in the same patent application, WO 02/32922, the obtaining of an alkylation product wherein the α/β ratio is equal to 2.5:1 is stated as an improvement.

In accordance with that reported in WO 02/32922, the synthesis can proceed as far as crude Fulvestrant by working with the α/β mixture, to then remove the undesired 7β product by simple crystallization.

Even if accepting this conclusion as correct, the fact remains that a percentage of about 30% of the final product, the β isomer, is a lost product and not recoverable in any way.

Therefore, the advantage that can be gained from a process which minimizes formation of the undesired β isomer is evident.

In international patent application WO 2005/077968, the starting product used is 3,17β-dihydroxyestra-1,3,5(10)-triene, which is protected in positions 3 and 17 as tetrahydropyranyl ether before being hydroxylated in position 6 to give 6-hydroxy-3,17β-di(tetrahydropyranyloxy)estra-1,3,5(10)-triene, by a reaction known in the literature and described in Synthesis December 1995, pages 1493-1495. Said reaction, as well as being considerably complex, requires a subsequent oxidation which transforms the hydroxyl in position 6 into a carbonyl group. In this respect, the function of the carbonyl group in position 6 is that of directing the alkylation in adjacent position 7 in a stereospecific manner, i.e. α.

The reagent used in Synthesis December 1995, pages 1493-1495, is an aqueous sodium hypochlorite solution giving a yield of 73%, whereas the reagent used in WO 2005/077968 is pyridinium chlorochromate giving a yield of 51%.

The pyridinium chlorochromate is a derivate of chromium VI, a notoriously carcinogenic compound whose industrial applicability appears to be problematic from the ecological and regulatory viewpoint.

The reaction yield from 3,17β-di(tetrahydropyranyloxy)estra-1,3,5(10)-triene to 3,17β-di(tetrahydropyranyloxy)-6-ketoestra-1,3,5(10)-triene is 43% according to the process described in WO 2005/077968 and 64% according to the process described in Synthesis December 1995, pages 1493-1495. No information is supplied in *Synthesis* on the subsequent alkylation in position 7 and on the decarbonylation in 6.

WO 2005/077968 describes the obtaining of 7α-[9-(4,4,5,5,5-pentafluorothiopentyl)nonyl]estra-1,3,5(10)-triene-3,17β-diol by means of two further reactions, with an overall yield of 34%.

International patent application WO 2006/015081 claims as inventive a process for obtaining intermediates usable for Fulvestrant synthesis with a 7-alkyl α to β epimer ratio such that 7α:7β = 12.1:1. This is an improvement on that described in WO02/32922 but there remain the problem of the β isomer, a non-recoverable lost product to be removed, and the need to aromatize the substrate after the first alkylation (step f, from intermediate 9341 to intermediate 9342).

The need is still felt to provide a new process for obtaining 7α-[9-(4,4,5,5,5-pentafluorothiopentyl)nonyl]estra-1,3,5(10)-triene-3,17β-diol of high purity which minimizes the disadvantages highlighted in the syntheses of the known art.

### SUMMARY OF THE INVENTION

The applicant has now established a new process enabling the industrial scale preparation of the product 7α-[9-(4,4,5,5,5-pentafluorothiopentyl)nonyl]estra-1,3,5(10)-triene-3,17β-diol at a high purity and with a highly stereoselective alkylation in position 7α, thus overcoming the disadvantages of the known art, for example by minimizing the recourse to chromatographic purifications, by eliminating the use of chromium VI compounds, and using as the starting product a steroid already aromatic in the first ring.

In particular, the quality of the intermediate 7α-[9-(4,4,5,5,5-pentafluorothiopentyl)nonyl]estra-1,3,5(10)-triene-3,17β-diol obtainable with the new process of the invention, is such as to enable pharmaceutical quality Fulvestrant to be obtained.

An aspect of the invention is therefore a process for preparing 7α-[9-(4,4,5,5,5-pentafluorothiopentyl)nonyl]estra-1,3,5(10)-triene-3,17β-diol of formula (I) 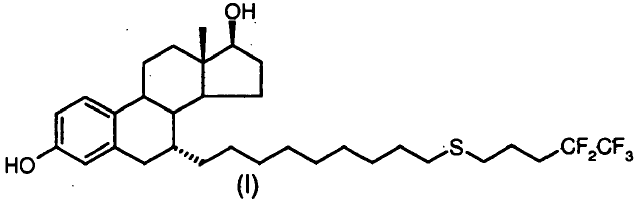 comprising the following steps:
a) protection of the hydroxyl groups of 3,17β-dihydroxy-estra-1,3,5(10)tetraen-6-one of formula (II) with 2-methoxypropene, to obtain the protected derivative of formula (III): 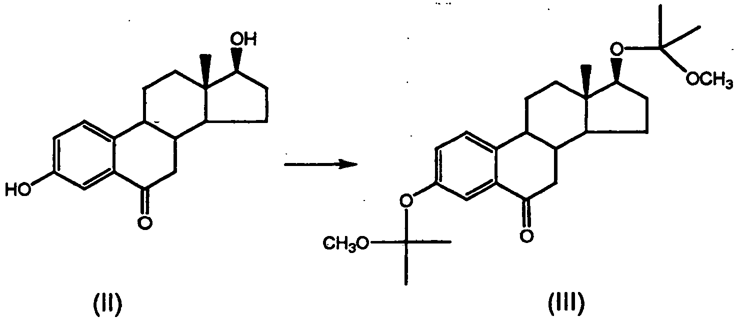
b) stereospecific alkylation in position 7α of the compound of formula (III) with 1,9-dibromononane, in a suitable solvent in the presence of a base, to obtain the alkylated derivative of formula (IV): 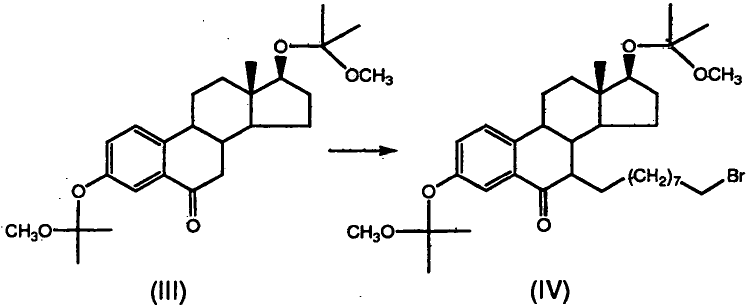
c) decarbonylation in position 6 or deprotection of the hydroxyl groups in positions 3 and 17 of the derivative of formula (IV), to obtain the compound of formula (V): 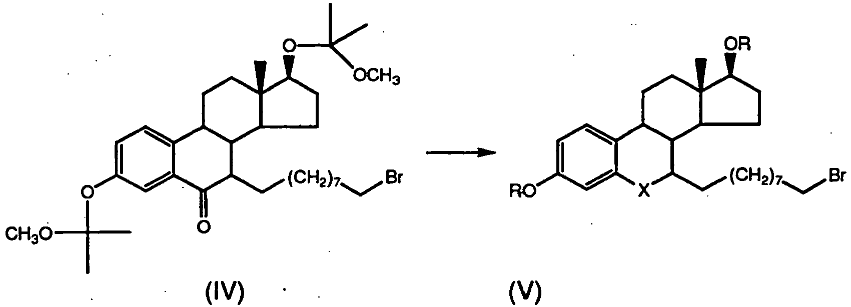 wherein R is 2-methoxypropyl and X is CH₂ if decarbonylation is carried out in position 6, or R is H and X is C=O if hydrolysis of the hydroxyl groups is carried out;
d) reaction of the compound of formula (V) with suitable S-alkylisothiouronium salts to obtain the compound of formula (VI) 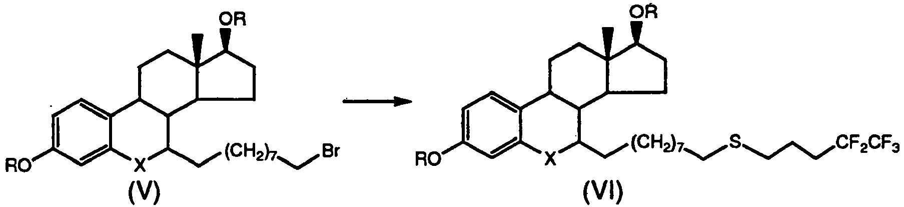 wherein R and X are as aforedefined;
e) deprotection of the hydroxyl groups in positions 3 and 17 or decarbonylation in position 6 of the compound of formula (VI) to obtain the desired product of formula (**I**) 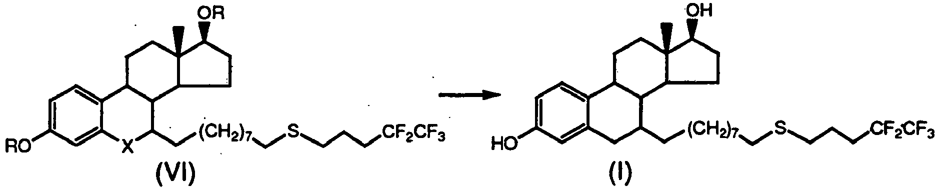 wherein R and X are as aforedefined.

The intermediates in the aforesaid process, having general formulas (IV), (V) and (VI) constitute a further aspect of the invention.

### BRIEF DESCRIPTION OF THE FIGURE

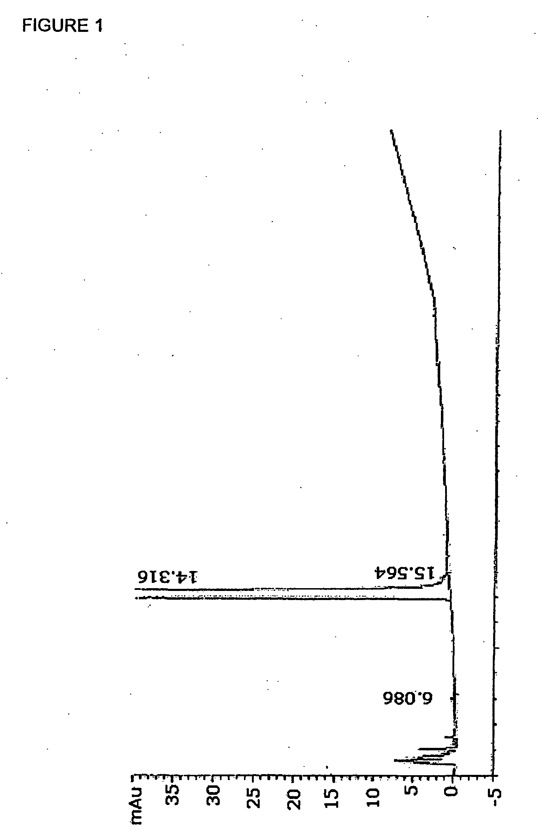

The features and advantages of the present process will be illustrated in detail in the following description and in the Figure 1, wherein the HPLC chromatographic profile of the product of Example 6 is shown.

### DETAILED DESCRIPTION OF THE INVENTION

The aforegiven starting product 3,17β-dihydroxy-estra-1,3,5(10)tetraen-6-one of formula (II), can for example be obtained by oxidation of 19-nortestosterone, a commercially available steroid which, when treated as described in Sferoids 66 (2001) 117-126 with pure oxygen flow at 120ºC in dimethylformamide in the presence of anhydrous potassium acetate, produces the product of formula (II). Said reaction on 19-nortestosterone is preferably conducted in pure dimethylformamide although a solvent chosen from dimethylsulphoxide, sulpholane, N-methylpyrrolidine, dimethylformamide and mixtures thereof can be used as the solvent.

In the same manner, the reaction temperature, being preferably higher than 100ºC, can be comprised between 50ºC and the boiling point of the reaction solvent or the solvent mixture.

The reaction is also conducted in the presence of a base, chosen for example from salts of alkali metals with weak acids or alcohols, preferably anhydrous potassium acetate; and in the presence of oxygen, introduced into the reaction environment as an air flow or, preferably, as a pure gas from a cylinder at a pressure between 1 and 25 bars.

In the process of the invention, the starting product of formula (II) is subjected to hydroxyl group protection in positions 3 and 17, with 2-methoxypropene, for example in accordance with the process known in the art and described in *"*Greene's Protective Groups in Organic Synthesis" 4th Ed., Wiley, page 77. Protection with 2-methoxypropene according to the invention has the advantages of a quantitative yield, low cost of the protecting reagent and simplicity of obtaining this latter; this is hence an improvement on that reported for example in Steroids 66 (2001) 117-126, wherein protection is carried out with t-butyldimethylsilyl chloride.

The present protection reaction is preferably conducted with 2-methoxypropene in the presence of POCl₃ as the catalyst, while the reaction solvent is preferably a solvent devoid of hydroxyl groups, chosen for example from the group consisting of toluene, xylene, methylene chloride, ethyl ether, dimethoxyethane, tetrahydrofuran, 2-methyl tetrahydrofuran, 3-methyl tetrahydrofuran, and mixtures thereof, being preferably methylene chloride. The reaction temperature is for example comprised between 0 and 40ºC, preferably between 20 and 40ºC.

The alkylation at step b) of the present process takes place in a highly stereospecific manner in position 7α by treating the product of formula (III) with 1,9-dibromononane as the alkylating agent, in a suitable solvent and in the presence of a base. The term "suitable solvent" means any non-alkylatable solvent resistant to reaction conditions, i.e. a non-protic solvent, chosen for example from dimethoxyethane, toluene, ethyl ether, tetrahydrofuran, 2-methyl tetrahydrofuran, 3-methyl tetrahydrofuran, and mixtures thereof. The preferred solvent is toluene.

The base used for the alkylation is for example chosen from the group consisting of potassium t-amylate, sodium t-amylate, potassium t-butylate, sodium t-butylate, potassium methylate, sodium methylate, lithium diisopropylamide (LDA), sodium amide, sodium hydride and potassium hydroxide in dimethylsulphoxide, hexamethyldisilazane salt of lithium or potassium. Preferably the base employed is potassium t-butylate.

The alkylation reaction at step b) is for example conducted at a temperature of between 50 ºC and the boiling point of the solvent or the solvent mixture used; preferably, the reaction is conducted at the boiling point of the solvent or solvent mixture.

According to a particularly preferred embodiment of the present process, step b) of alkylation is conducted in the presence of small quantities of potassium iodide, which facilitate the reaction.

The alkylated product of formula (IV) is obtained with a yield of about 80% and high purity. An optional purification of the alkylated product, before being subjected to the next step, can be carried out by a simple chromatographic purification on silica gel.

The introduction of a fluorinated chain can take place according to the present process using S-alkylisothiouronium salts on the alkylated product of formula (IV), previously subjected to decarbonylation in position 6, or to hydrolysis with deprotection of hydroxyl groups in positions 3 and 17, to obtain the product of the aforesaid formula (V).

After introduction of the fluorinated chain at step e) of the present process, the product of formula (VI) is subjected to decarbonylation in position 6 or to hydrolysis with deprotection of hydroxyl groups in positions 3 and 17, depending on the fact that at step c) a hydrolysis has been conducted with deprotection of the hydroxyl groups in 3 and 17 or a decarbonylation in 6 respectively.

The decarbonylation reaction in position 6 which can be conducted at step c) or at step e) of the present process, is preferably conducted at a temperature of between 0 and 40ºC by reacting the product of formula (IV) or of formula (VI) respectively, with triethylsilane in methylene chloride in the presence of boron trifluoride etherate.

When the decarbonylation is conducted at step c) of the present process, at the end of the aforesaid reaction with triethylsilane in methylene chloride, the product may be treated again to remedy even a partial hydrolysis of the protective groups, without having isolation, but instead directly in anhydrous solution of methylene chloride, with POCl₃ and 2-methoxypropene, before undergoing the next step d) of fluorinated chain introduction.

It was nevertheless surprisingly found that the decarbonylation reaction at step c) takes place even on the partially or totally deprotected product with similarly good results.

Said step d) is conducted with the use of suitable S-alkylisothiouronium salts, these being products that can be prepared according to general instructions given in Vogel "Chimica Organic" 2nd edition. Ambrosiana Publishing House. The preferred reagent used in step d) of the present process is carbamimidothioic acid 4,4,5,5,5-pentafluoropentyl mesylate ester, having the following formula: 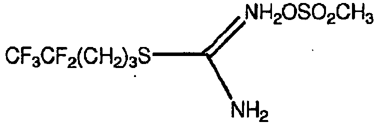 in the presence of sodium hydroxide and water.

Said reaction at step d) of the present process is conducted at a temperature comprised between 55 and 75ºC in a non-alkylatable water-miscible solvent such as acetonitrile, in the presence of a base such as triethylamine.

Deprotection of the hydroxyl groups in positions 3 and 17 can be conducted at step c) or at step e) of the present process and is preferably conducted at a temperature comprised between 5 and 40ºC, by treating the product of formula (IV) or formula (VI), respectively, with aqueous solutions of an acid salt or a protic acid having a pH less than or equal to 4; the reaction is preferably conducted in a biphasic aqueous acid/methylene chloride solution environment.

According to a particularly preferred embodiment of the present process, the reactions at steps a), b) and c) and the decarbonylation reaction at step e) are conducted in an inert argon or nitrogen atmosphere. Moreover, said reactions are preferably carried out under conditions such as to minimize the presence of water in the reaction environment.

The following non-limiting examples of the present invention are given by way of illustration.

### Example 1

### Preparation of the compound of formula (III) - Step a)

10 g of 19-nortestosterone (Nandrolone) are loaded into an autoclave with 100 ml of dimethylformamide and 5.4 g of anhydrous potassium acetate at ambient temperature.

The autoclave pressure is brought to 8 bar with pure oxygen then the temperature is brought to 120ºC for 12 hours.

At the end of the reaction, monitored by TLC with 1/1 heptane/acetone, the reaction solution is poured into 200 ml of water, treated with carbon and the product is extracted with ethyl acetate.

The organic phase, washed with water and dried over sodium sulphate, provides, after evaporation of the solvent under reduced pressure, 4.8 g of product which is found to be the aforestated 3,17β-dihydroxy-estra-1,3,5(10)trien-6-one of formula (II), of a suitable quality for the continuation of the synthesis.

On the thus obtained product, after purification by chromatography for analytical purposes (silica gel, heptane/ethyl acetate), ¹H-NMR and mass spectroscopy analyses were carried out and the following results were obtained:
Mass by electron impact: [M⁺]= 286
¹H-NMR (500 MHz, CDCl₃): δ (ppm) 0.80 (s, 3H, 13-CH₃); 3.76 (t J=8 Hz, 1H, 17-H); 7.04 (dd J=8Hz, J= 3Hz, 2-H); 7.30 (d J= 8Hz, 2H); 7.50 (d J=2Hz, 1H) aromatic protons
30 g of the product obtained as above are suspended in 300 ml of methylene chloride. 110 ml of 2-methoxypropene and 0.05 ml of POCl₃ are added and the mixture is refluxed (T=35ºC) for about 4 hours.

At the end of the reaction, monitored by TLC with 1/1 heptane/acetone, 10 ml of triethylamine are added and the entire mixture is unloaded into 1 litre of ice-water-Na₂CO₃. The product is extracted with methylene chloride.

The organic phase, basified with pyridine and washed with water, is evaporated under reduced pressure.

After filtration from heptane and drying under reduced pressure, 34 g of the title product are obtained.

Following chromatographic purification for analytical purposes, the product obtained in this manner was subjected to ¹H-NMR analysis and the following result was obtained:
¹H-NMR (500 MHz, CDCl₃): δ (ppm) 0.78 (s, 3H, 13-methyl); 1.30 (d, 6H, 17-protection); 1.45 (d, 6H, 3-protection); 3.20 (s, 3H, CH₃O- 17-protection); 3.40 (s, 3H, CH₃O- 3-protection); 3.70 (t, J= 5Hz, 1H, H₁₇); 7.25 (dd, J=8Hz, 3Hz, 1 H, H₂); 7.29 (d J= 8Hz, 1 H, H₁); 7.72 (d J= 3Hz, 1 H, H₄)

### EXAMPLE 2

### Preparation of the compound of formula (IV) - Step b)

18 g of the product of formula (III) prepared as described in example 1 are dissolved in 300 ml of anhydrous toluene at 20/25ºC.

5.7 g of potassium tert-butylate are added to the solution obtained and heated to 50ºC for 1 hour.

The temperature is returned to 10ºC, 0.7 g of potassium iodide and 34 ml of 1,9-dibromononane are added.

The temperature is returned to 100ºC and is maintained for about three hours with additions of potassium tert-butylate.

At the end of the reaction, monitored by TLC with 1/1 heptane/acetone, the mixture is poured into water and the product is extracted with ethyl acetate.

The organic phase, evaporated under reduced pressure, provides 60 g of a semisolid product which is purified by chromatography (silica gel, toluene/acetone).

After evaporation of the fractions containing the product of formula (IV), 12 g of said product are obtained.

### EXAMPLE 3

### Preparation of the compound of formula (V) in which R is 2-methoxypropyl and X is CH₂ - Step c) of decarbonylation in position 6

7.5 g of the product of formula (V) prepared as aforedescribed in example 2, are suspended in 250 ml of anhydrous methylene chloride at 0ºC. 58 ml of triethylsilane Et₃SiH and 74 ml of BF₃.Et₂O are added and left under agitation for about 4 hours allowing the temperature to rise slowly to 20/25ºC.

At the end of the reaction, monitored by TLC with 8/2 toluene/acetone, the mixture is poured into 1 litre of water/NaHCO₃ then extracted with methylene chloride. The organic phase is washed with water, dried over sodium sulphate and evaporated under reduced pressure.

After filtration from heptane and drying under reduced pressure, 7g of the product of the title are obtained, of a suitable quality for continuation of the synthesis. Following chromatographic purification for analytical purposes, the product obtained in this manner was subjected to ¹H-NMR analysis and the following result was obtained:
¹H-NMR (500 MHz, CDCl₃): δ (ppm) 0.82 (s, 3H, 13-methyl); 1.36 (s, 6H, 17-protection); 1.50 (s, 6H, 3-protection); 3.25 (s, 3H, MeO- 17-protection); 3.42 (m, 2H, -CH₂-Br); 3.44 (s, 3H, MeO- 17-protection); 3.75 (t J= 8Hz, 1 H, H₁₇); 6.80 (d J=2Hz, 1 H, H₄); 6.93 (dd J= 2 and 8Hz, 1 H, H₂); 7.18 (d J=8Hz, 1 H, H₁)

### EXAMPLE 4

### Preparation of the compound of formula (VI) in which R is 2-methoxypropyl and X is CH₂ - Step d)

9 g of the product of formula (V) in which R is 2-methoxypropyl and X is CH₂ prepared as aforedescribed in example 3, are suspended in a 150/1.5 ml acetonitrile/triethylamine solvent mixture.

5.8 g of carbamimidothioic acid 4,4,5,5,5-pentafluoropentyl mesylate ester prepared as described later in example 7 are then added, dissolved in 20 ml of water and 3.5 g of NaOH, again as an aqueous solution.

The suspension is brought to 70ºC for about 3 hours.

At the end of the reaction, monitored by TLC with 9/1 heptane/ethyl acetate, the mixture is poured into 100 ml of water then extracted with toluene/pyridine.

After evaporation of the solvent under reduced pressure, 11 g of the product of the title are obtained, directly usable for the next reaction after TLC analysis.

During evaporation of the solvent and all the work-up steps, pyridine is used to prevent hydrolysis of the protective groups in positions 3 and 17.

### EXAMPLE 5

### Preparation of the compound of formula (1) Step e) of hydrolysis with deprotection of the hydroxyl groups in positions 3 and 17.

The product of formula (VI) in which R is 2-methoxypropyl and X is CH₂-obtained as aforedescribed in example 4, is dissolved in a 400/200 ml biphasic methylene chloride/water system.

8.5 g of NaHSO₄.H₂O are added and the mixture is refluxed under agitation for 3 hours.

At the end of the reaction, monitored by TLC with 8/2 toluene/acetone, the reaction is neutralized with an aqueous solution of NaHCO₃ and the product is extracted with methylene chloride.

The organic phase is evaporated under reduced pressure.

The product obtained in this manner, after chromatographic purification on silica gel using 9/1 toluene/acetone as eluent and drying under reduced pressure provides 4.8 g of the product of the title.

On a sample of this product ¹H-NMR and mass spectroscopic analyses were carried out and the following results were obtained.
Mass by electron impact: [M⁺] = 590
¹H-NMR (500 MHz, CDCl₃): δ (ppm) 0.80 (s, 3H, 13 -CH3); 0.9-3.0 (Σm, aliphatic protons); 2.52 (t, J= 8 Hz, 2H, -CH₂SCH₂-) and 2.62 (t, J= 8 Hz, 2H, -CH₂SCH₂-); 3.78 (m, 1 H, H17); 4.82 (s, 1H, -OH phenol); 6.58 (d, J= 1 Hz, 1 H, H4); 6.68 (dd, J= 8 Hz, J= 1 Hz, 1H, H2); 7.20 (d, J= 8 Hz, 1H, H1)

### EXAMPLE 6

### Preparation of Fulvestrant

4.5 g of the product of formula (I) obtained as described above in example 5 are dissolved in 60 ml of methylene chloride at 0/5ºC.

1.3 g of m-chloroperbenzoic acid are added in portions over 2 hours reaction time. At the end of the reaction, monitored by TLC with 7/3 toluene/acetone, the mixture is poured into a 1 litre/15 g/30 g water/Na₂SO₃/NaHCO₃ solution and agitated at ambient temperature for about 15 minutes.

The mixture is extracted with methylene chloride from which, after solvent evaporation, 4 g of an off-white solid are obtained.

The solid is chromatographed over silica gel using as eluent a 7:3 heptane/acetone mixture and crystallized with ethyl acetate at least twice.

After drying under reduced pressure, 2.35 g of Fulvestrant are obtained whose ¹H-NMR and mass spectroscopic analyses gave results in agreement with those reported in the literature and the HPLC chromatographic profile shown in Figure 1, in which the impurities found have a percentage area of less than 0.030%.

### EXAMPLE 7

### Preparation of carbamimidothioic acid 4,4,5,5,5-pentafluoropenlyl mesylate ester

20 g of pentafluoropentanol and 21 ml of triethylamine are dissolved in 400 ml of methylene chloride at ambient temperature under inert atmosphere.

The mixture is cooled to 0/5ºC, 9.7 ml of mesyl chloride are added followed by agitation in a cold bath for about 2 hours.

At the end of the reaction, monitored by TLC with 7/3 toluene/ethyl acetate, 100 ml of water are added and the mixture extracted with 200 ml of methylene chloride. The solvent is evaporated under reduced pressure to obtain 27 g of 4,4,5,5,5-pentafluoropentanol mesylate as an oily product, which is used as such.

19 g of this product are dissolved in 100 ml of isopropanol, 5.7 g of thiourea are added and the mixture is refluxed for about 10 hours under inert atmosphere. The solvent is removed under reduced pressure and 25 g of carbamimidothioic acid 4,4,5,5,5-pentafluoropentyl mesylate ester are crystallized, firstly from tetrahydrofuran (65 ml) washing the crystallized product with isopropyl ether, then from acetonitrile (70 ml).

After drying under reduced pressure, 17 g of the product of the title are obtained which showed the following NMR spectrum.
¹H-NMR (500 MHz, DMSO-d₆): δ (ppm) 1.85 (m, 2H, -CH₂-); 2.37 (m, 2H, -CH₂-); 2.40 (s, 3H, -CH₃); 3.22 (t J= 8Hz, 2H, -CH₂-); 9.15 (s, 4H, protons on heteroatom)

### EXAMPLE 8

### Preparation of the product of formula (I) starting from the product of formula (IV) - From Step c) in which deprotection of the hydroxyl groups in 3 and 17 is carried out, to Step e) in which decarbonylation in 6 is carried out

26 g of the product of formula (IV) prepared as described in example 2, are dissolved at ambient temperature in 400 ml of methylene chloride and 200 ml of water.

17 g of NaHSO₄ H₂O are added and the mixture agitated for 3 hours at 35ºC.

At the end of the reaction, monitored by TLC with 8/2 toluene/acetone, the mixture is neutralized with 15 g of NaHCO₃ and extracted with methylene chloride.

After drying with sodium sulphate, the solvent is removed under reduced pressure and the residue treated with heptane.

The product is filtered off and dried at 40ºC under reduced pressure for 8 hours to obtain 20 g of the product of formula (V) in which X is C=O and R is H, of a quality suitable for continuation of the synthesis. On a sample of this product, purified for analytical purposes, ¹H-NMR spectroscopic analyses were carried out and the following results were obtained.
1 H-NMR (500 MHz, CDCl3): δ (ppm) 0.76 (s, 3H, 13 -CH3); 3.38 (t, J= 6 Hz, 2H, - CH₂-Br); 3.75 (t, J= 6 Hz, 1 H, H17); 5.5 (broad signal, 1 H, -OH17); 7.02 (dd, J= 8 Hz, J= 1 Hz, 1 H, H2); 7.27 (d, J= 8 Hz, 1 H, H1); 7.52 (d, J= 1 Hz, 1 H, H4).

The mass spectrum, obtained by electron impact, showed [M⁺] = 492 and [M⁺] 7α chain = 286.

This product is reacted using a procedure similar to that aforedescribed in example 4, to obtain 24 g of the product of formula (VI) in which X is C=O and R is H, found to be directly usable for the next reaction after TLC analysis.

On a sample of this product, purified for analytical purposes, ¹H-NMR spectroscopic analyses were carried out and the following results were obtained.
¹H-NMR (500 MHz, CDCl₃): δ (ppm) 0.82 (s, 3H, 13 -CH₃); 3.77 (t, J= 8 Hz, 1H, H₁₇); 7.05 (dd, J= 8 Hz, J= Hz, 1H, H₂); 7.27 (d, J= 8 Hz, 1H, H₁); 7.55 (d, J= 1 Hz, 1H, H₄).

The mass spectrum, obtained by electron impact, showed [M⁺] = 604 and [M⁺] - 7α chain = 286.

This product is then reacted using a procedure similar to that aforedescribed in example 3, to obtain 18.1 g of the product of formula (I), purifiable as described in the preceding examples.

## Claims

1. Process for preparing 7α-[9-(4,4,5,5,5-pentafluorothiopentyl)nonyl]estra-1,3,5(10)-triene-3,17β-diol of formula (I) 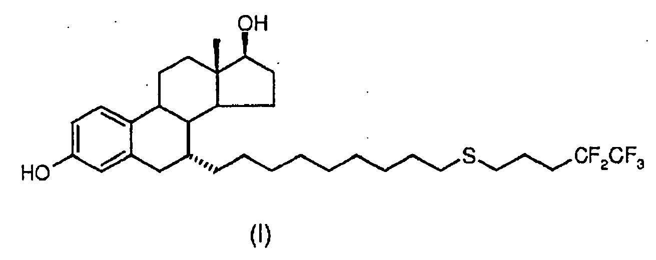 comprising the following steps:
a) protection of the hydroxyl groups of 3,17β-dihydroxy-estra-1,3,5(10)tetraen-6-one of formula (II) with 2-methoxypropene, to obtain the protected derivative of formula (III): 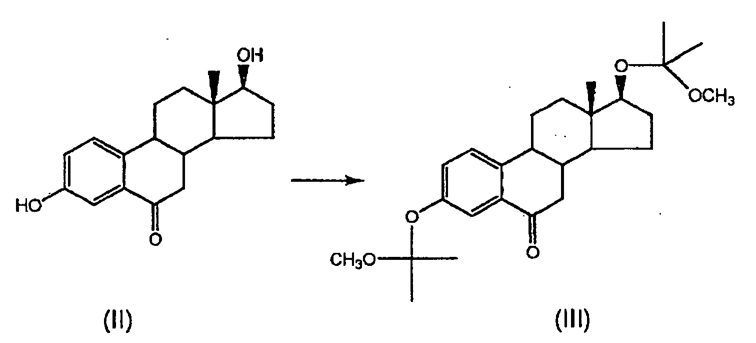
b) stereospecific alkylation in position 7α of the compound of formula (III) with 1,9-dibromononane, in a suitable solvent in the presence of a base, to obtain the alkylated derivative of formula (IV): 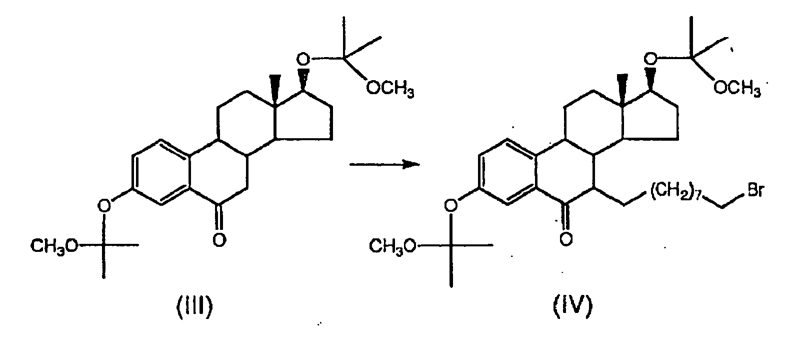
c) decarbonylation in position 6 or deprotection of the hydroxyl groups in positions 3 and 17 of the derivative of formula (IV), to obtain the compound of formula (V): 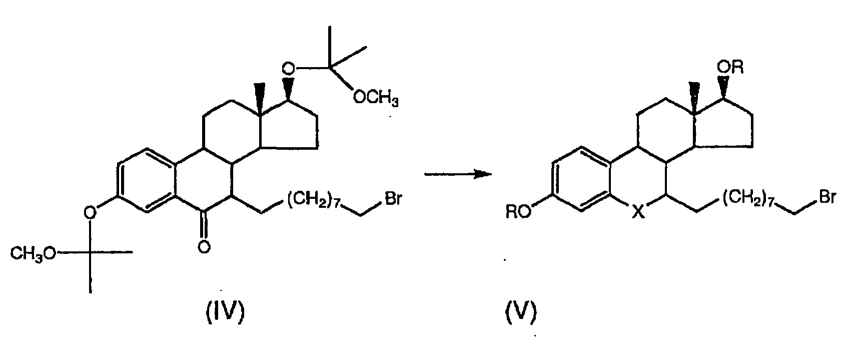 wherein R is 2-methoxypropyl and X is CH₂ if the decarbonylation is carried out in position 6, otherwise R is H and X is C=O if hydrolysis of the hydroxyl groups is carried out;
d) reaction of the compound of formula (V) with suitable S-alkylisothiouronium salts to obtain the compound of formula (VI) 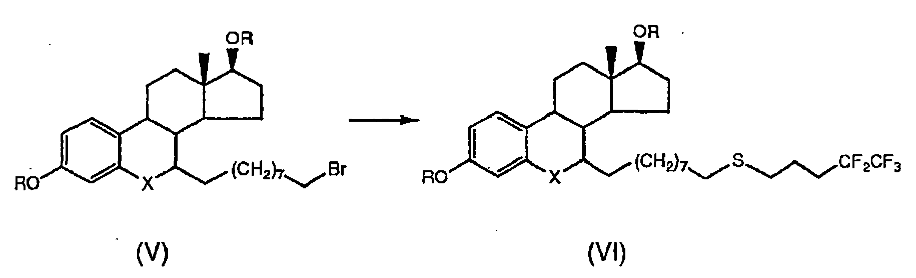 wherein R and X are as aforedefined;
e) deprotection of the hydroxyl groups in positions 3 and 17 or decarbonylation in position 6 of the compound of formula (VI) to obtain the desired product of formula (I) 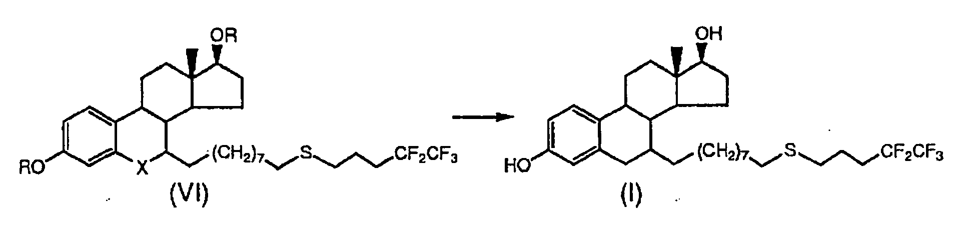 wherein R and X are as aforedefined.

2. Process according to claim 1 wherein the protection reaction at step a) is conducted with 2-methoxypropene in the presence of POCl₃ as catalyst, in a solvent devoid of hydroxyl groups, at a temperature comprised between 0 and 40ºC.

3. Process according to claim 2 wherein said solvent is chosen from the group consisting of toluene, xylene, methylene chloride, ethyl ether, dimethoxyethane, tetrahydrofuran, 2-methyl tetrahydrofuran, 3-methyl tetrahydrofuran, and mixtures thereof.

4. Process according to claim 1 wherein at step b) of alkylation said solvent is a solvent chosen from dimethoxyethane, toluene, ethyl ether, tetrahydrofuran and mixtures thereof.

5. Process according to claim 1 wherein at step b) of alkylation said base is chosen from the group consisting of potassium t-amylate, sodium t-amylate, potassium t-butylate, sodium t-butylate, potassium methylate, sodium methyate, lithium diisopropylamide (LDA), sodium amide, sodium hydride and potassium hydroxide in dimethylsulphoxide and hexamethyldisilazane salt of lithium or potassium.

6. Process according to claim 1 wherein said alkylation at step b) is conducted at a temperature comprised between 50ºC and the boiling point of the solvent used or the solvent mixture.

7. Process according to claim 6 wherein said alkylation at step b) is conducted at the boiling point of the solvent or solvent mixture.

8. Process according to claim 1 wherein step b) of alkylation is conducted in the presence of potassium iodide in a quantity equal to about 5% by weight of the compound of formula (III).

9. Process according to claim 1 wherein said decarbonylation reaction in position 6 at step c) or at step e) is conducted at a temperature comprised between 0 and 40ºC by reacting the product of formula (IV) or of formula (VI) respectively with triethylsilane in methylene chloride in the presence of borotrifluoride etherate.

10. Process according to claim 1 wherein said S-alkylisothiouronium salt of step d) is carbamimidothioic acid 4,4,5,5,5-pentafluoropentyl mesylate ester, in the presence of sodium hydroxide and water.

11. Process according to claim 1 wherein said step d) is conducted at a temperature comprised between 55 and 75ºC, in a non-alkylatable water-miscible solvent in the presence of a base.

12. Process according to claim 11 wherein said solvent is acetonitrile and said base is triethylamine.

13. Process according to claim 1 wherein said deprotection reaction of hydroxyl groups in positions 3 and 17 at step c) or at step e), is conducted at a temperature comprised between 5 and 40ºC, by treatment of the product of formula (IV) or formula (VI) respectively, with aqueous solutions of an acid salt or a protic acid, having a pH less than or equal to 4.

14. Process according to claim 13 wherein said reaction is conducted in a biphasic aqueous acid/methylene chloride solution environment.

15. Process according to claim 1 wherein the reactions of steps a), b) and c) and the decarbonylation reaction of step e) are conducted in an inert argon or nitrogen atmosphere.

16. Process according to claim 1 wherein the reactions of steps a), b) and c) and the decarbonylation reaction of step e) are conducted under conditions such as to minimize the presence of water in the reaction environment.

17. Products of general formula (IV), (V) and (VI) as intermediates in the process as defined in claims 1-16.

## Patentansprüche

1. Prozess zum Herstellen von 7α-[9-(4,4,5,5,5-Pentafluorthiopentyl)nonyl]estra-1,3,5(10)-trien-3,17β-diol der Formel (I) 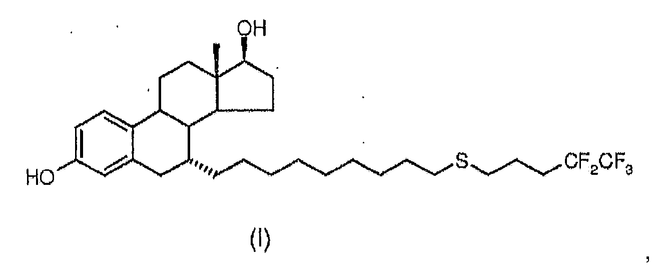 der die folgenden Schritte umfasst:
a) Schützung der Hydroxylgruppen von 3,17β-Dihydroxy-estra-1,3,5(10)tetraen-6-on der Formel (II) mit 2-Methoxypropen, um das geschützte Derivat der Formel (III) zu erhalten: 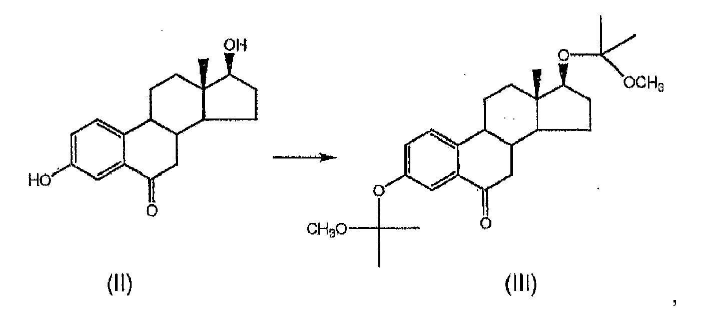
b) Stereospezifische Alkylierung in der Position 7α der Verbindung der Formel (III) mit 1,9-Dibromnonan in einem geeigneten Lösungsmittel in der Gegenwart einer Base, um das alkylierte Derivat der Formel (IV) zu erhalten: 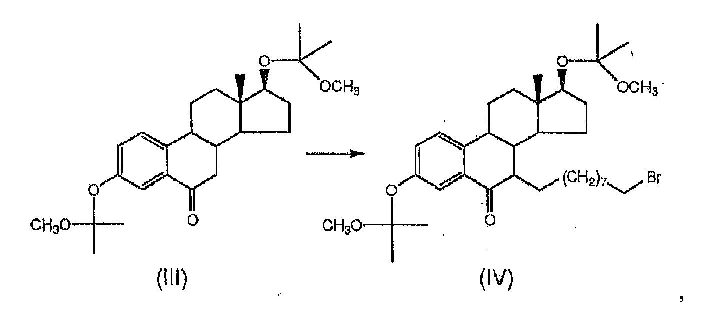
c) Decarbonylierung in der Position 6 oder Entschützung der Hydroxylgruppen in den Positionen 3 und 17 des Derivats der Formel (IV), um die Verbindung der Formel (V) zu erhalten: 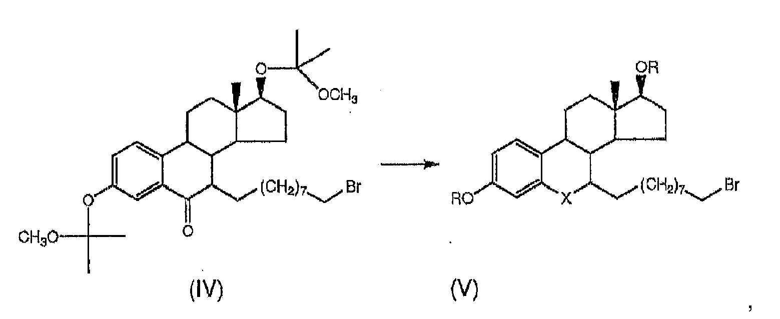 wobei R 2-Methoxypropyl ist und X CH₂ ist, wenn die Decarbonylierung in der Position 6 ausgeführt wird, ansonsten R H ist und X C=O ist, wenn die Hydrolyse der Hydroxylgruppen ausgeführt wird,
d) Reaktion der Verbindung der Formel (V) mit geeigneten S-Alkylisothiouroniumsalzen, um die Verbindung der Formel (VI) zu erhalten: 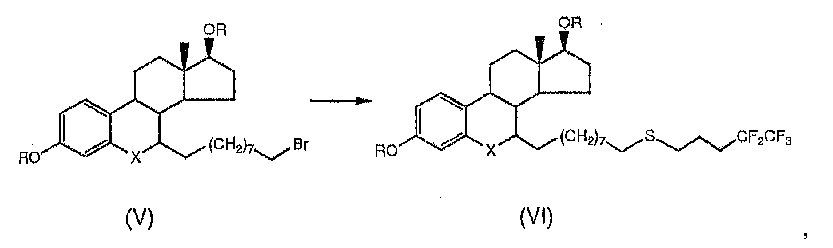 wobei R und X wie zuvor definiert sind,
e) Entschützung der Hydroxylgruppen in den Positionen 3 und 17 oder Decarbonylierung in der Position 6 der Verbindung der Formel (VI), um das gewünschte Produkt der Formel (I) zu erhalten: 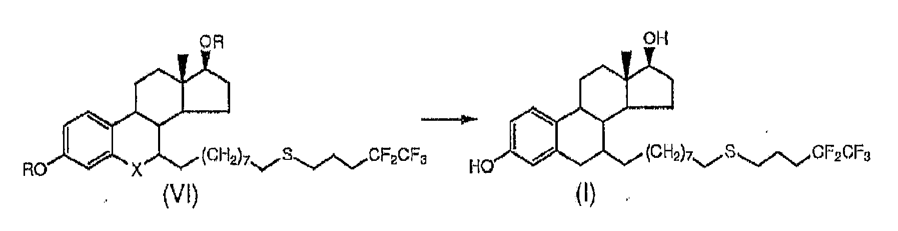 wobei R und X wie zuvor definiert sind.

2. Prozess nach Anspruch 1, wobei die Schützungsreaktion im Schritt a) mit 2-Methoxypropen in der Gegenwart von POCl₃ als Katalysator in einem Lösungsmittel ohne Hydroxylgruppen bei einer Temperatur zwischen 0 und 40°C durchgeführt wird.

3. Prozess nach Anspruch 2, wobei das Lösungsmittel aus der Gruppe ausgewählt wird, die aus Toluol, Xylol, Methylenchlorid, Ethylether, Dimethoxyethan, Tetrahydrofuran, 2-Methyltetrahydrofuran, 3-Methyltetrahydrofuran und Mischungen davon besteht.

4. Prozess nach Anspruch 1, wobei im Schritt b) der Alkylierung das Lösungsmittel ein Lösungsmittel ist, das aus Dimethoxyethan, Toluol, Ethylether, Tetrahydrofuran und Mischungen davon ausgewählt wird.

5. Prozess nach Anspruch 1, wobei im Schritt b) der Alkylierung die Base aus der Gruppe ausgewählt wird, die aus Kalium-t-amylat, Natrium-t-amylat, Kalium-t-butylat, Natrium-t-butylat, Kaliummethylat, Natriummethylat, Lithiumdiisopropylamid (LDA), Natriumamid, Natriumhydrid und Kaliumhydroxid in Dimethylsulfoxid und dem Hexamethyldisilazansalz von Lithium oder Kalium besteht.

6. Prozess nach Anspruch 1, wobei die Alkylierung im Schritt b) bei einer Temperatur zwischen 50°C und dem Siedepunkt des verwendeten Lösungsmittels oder der Lösungsmittelmischung durchgeführt wird.

7. Prozess nach Anspruch 6, wobei die Alkylierung im Schritt b) an dem Siedepunkt des Lösungsmittels oder der Lösungsmittelmischung durchgeführt wird.

8. Prozess nach Anspruch 1, wobei der Schritt b) der Alkylierung in der Gegenwart von Kaliumiodid in einer Menge gleich zu etwa 5 Gewichts-% der Verbindung der Formel (III) durchgeführt wird.

9. Prozess nach Anspruch 1, wobei die Decarbonylierungsreaktion in der Position 6 im Schritt c) oder im Schritt e) bei einer Temperatur zwischen 0 und 40°C jeweils durch Reagieren des Produkts der Formel (IV) oder der Formel (VI) mit Triethylsilan in Methylenchlorid in der Gegenwart von Bortrifluorid-Etherat durchgeführt wird.

10. Prozess nach Anspruch 1, wobei das S-Alkylisothiouroniumsalz des Schritts d) Carbamimidothionsäure-4,4,5,5,5-pentafluorpentylmesylatester in der Gegenwart von Natriumhydroxid und Wasser ist.

11. Prozess nach Anspruch 1, wobei der Schritt d) bei einer Temperatur zwischen 55 und 75°C in einem nicht-alkylierbaren wassermischbaren Lösungsmittel in der Gegenwart einer Base durchgeführt wird.

12. Prozess nach Anspruch 11, wobei das Lösungsmittel Acetonitril ist und die Base Triethylamin ist.

13. Prozess nach Anspruch 1, wobei die Entschützungsreaktion der Hydroxylgruppen in den Positionen 3 und 17 im Schritt c) oder im Schritt e) bei einer Temperatur zwischen 5 und 40°C jeweils durch Behandlung des Produkts der Formel (IV) oder Formel (VI) mit wässrigen Lösungen eines sauren Salzes oder einer protischen Säure mit einem pH von weniger als oder gleich zu 4 durchgeführt wird.

14. Prozess nach Anspruch 13, wobei die Reaktion in einer zweiphasigen Lösungsumgebung mit wässriger Säure/Methylenchlorid durchgeführt wird.

15. Prozess nach Anspruch 1, wobei die Reaktionen der Schritte a), b) und c) und die Decarbonylierungsreaktion des Schritts e) in einer inerten Argon- oder Stickstoffatmosphäre durchgeführt werden.

16. Prozess nach Anspruch 1, wobei die Reaktionen der Schritte a), b) und c) und die Decarbonylierungsreaktion des Schritts e) unter Bedingungen durchgeführt werden, wie um die Gegenwart von Wasser in der Reaktionsumgebung zu minimieren.

17. Produkte der allgemeinen Formel (IV), (V) und (VI) als Zwischenprodukte in dem Prozess wie in den Ansprüchen 1-16 definiert.

## Revendications

1. Procédé de préparation de 7α-[9-(4,4,5,5,5-pentafluorothiopentyl)nonyl]estra-1,3,5(10)-triène-3,17β-diol de formule (I) 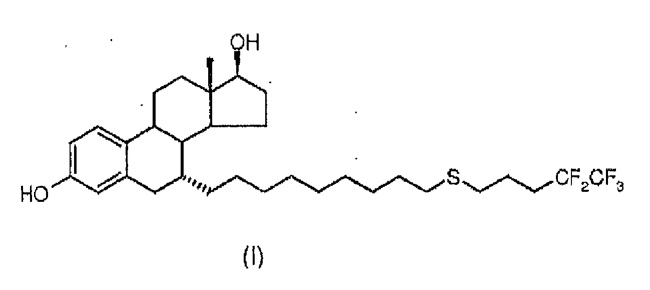 comprenant les étapes suivantes :
a) protection des groupes hydroxyles de 3,17β-dihydroxy-estra-1,3,5(10)tetraén-6-one de formule (II) avec du 2-méthoxypropène, pour obtenir le dérivé protégé de formule (III) : 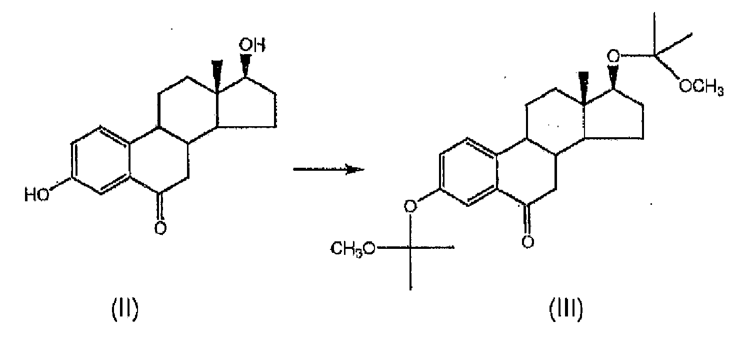
b) alkylation stéréospécifique en position 7α du composé de formule (III) avec du 1,9-dibromononane, dans un solvant adéquat en présence d'une base, pour obtenir le dérivé alkylé de formule (IV) : 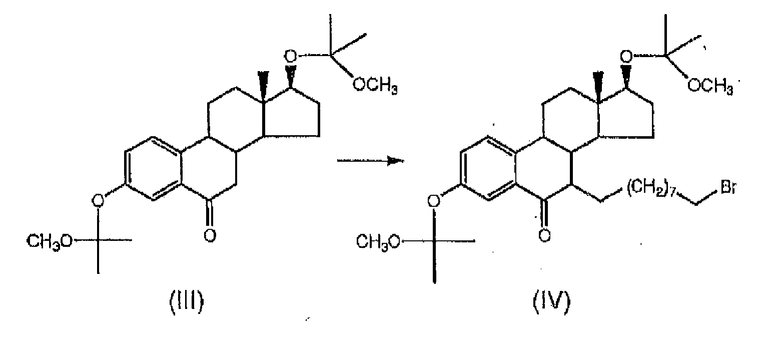
c) décarbonylation en position 6 ou déprotection des groupes hydroxyles en positions 3 et 17 du dérivé de formule (IV), pour obtenir le composé de formule (V) : 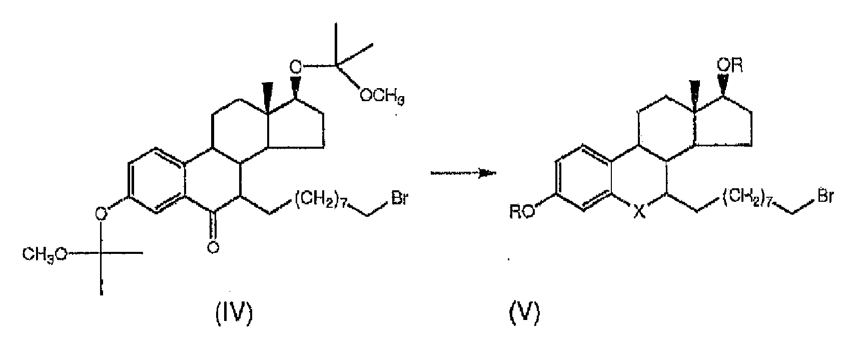 dans laquelle R représente un groupe 2-méthoxypropyle et X représente un groupe CH₂ si la décarbonylation est réalisée en position 6, sinon R représente un atome d'H et X représente C=O si l'hydrolyse des groupes hydroxyles est réalisée ;
d) réaction du composé de formule (V) avec des sels S-alkylisothiouronium adéquats pour obtenir le composé de formule (VI) 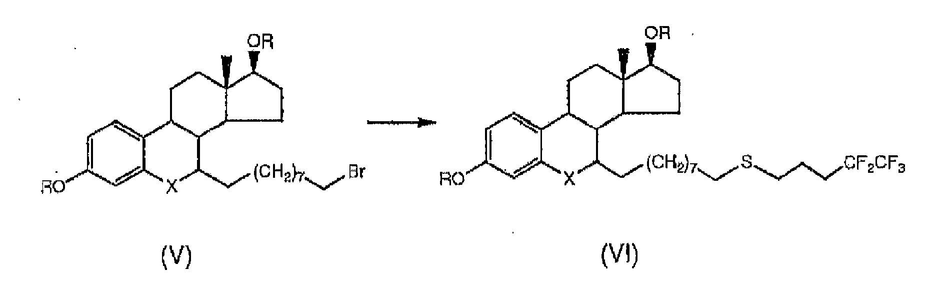 dans laquelle R et X sont tels que définis ci-dessus ;
e) déprotection des groupes hydroxyles en positions 3 et 17 ou décarbonylation en position 6 du composé de formule (VI) pour obtenir le produit souhaité de formule (I) 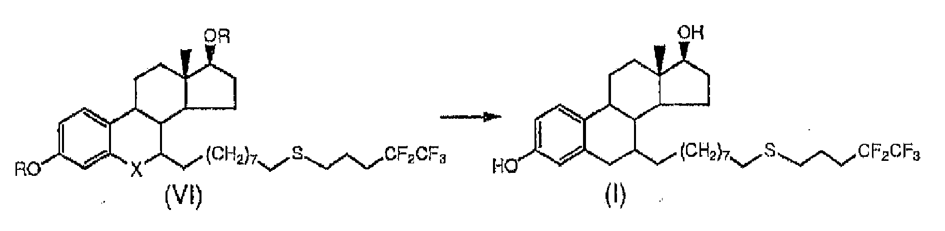 dans laquelle R et X sont tels que définis ci-dessus.

2. Procédé selon la revendication 1 dans lequel la réaction de protection lors de l'étape a) est effectuée avec du 2-méthoxypropèneen présence de POCl₃ en tant que catalyseur, dans un solvant exempt de groupes hydroxyles, à une température comprise entre 0 et 40 °C.

3. Procédé selon la revendication 2 dans lequel ledit solvant est choisi dans le groupe constitué du toluène, du xylène, du chlorure de méthylène, de l'éther éthylique, du diméthoxyéthane, du tétrahydrofuranne, du 2-méthyltétrahydrofuranne, du 3-méthyltétrahydrofuranne et de leurs mélanges.

4. Procédé selon la revendication 1 dans lequel lors de l'étape b) d'alkylation ledit solvant est un solvant choisi parmi le diméthoxyéthane, le toluène, l'éther éthylique, le tétrahydrofuranne et leurs mélanges.

5. Procédé selon la revendication 1 dans lequel lors de l'étape b) d'alkylation ladite base est choisie dans le groupe constitué du t-amylate de potassium, du t-amylate de sodium, du t-butylate de potassium, du t-butylate de sodium, du méthylate de potassium, du méthylate de sodium, de la lithium-diisopropylamide (LDA), de l'amidure de sodium, de l'hydrure de sodium et de l'hydroxyde de potassium dans un sel diméthylsulfoxyde et hexaméthyldisilazane de lithium ou de potassium.

6. Procédé selon la revendication 1 dans lequel ladite alkylation lors de l'étape b) est effectuée à une température comprise entre 50 °C et le point d'ébullition du solvant utilisé ou du mélange de solvants.

7. Procédé selon la revendication 6 dans lequel ladite alkylation lors de l'étape b) est effectuée au point d'ébullition du solvant ou du mélange de solvants.

8. Procédé selon la revendication 1 dans lequel l'étape b) d'alkylation est effectuée en présence d'iodure de potassium en une quantité égale à environ 5 % en poids du composé de formule (III).

9. Procédé selon la revendication 1 dans lequel ladite réaction de décarbonylation en position 6 lors de l'étape c) ou lors de l'étape e) est effectuée à une température comprise entre 0 et 40 °C en faisant réagir le produitde formule (IV) ou de formule (VI) respectivement avec du triéthylsilane dans du chlorure de méthylène en présence d'éthérate de borotrifluorure.

10. Procédé selon la revendication 1 dans lequel ledit sel S-alkylisothiouronium de l'étape d) est un ester de 4,4,5,5,5-pentafluoropentyl-mésylate d'acide carbamimidothioïque, en présence d'hydroxyde de sodium et d'eau.

11. Procédé selon la revendication 1 dans lequel ladite étape d) est effectuée à une température comprise entre 55 et 75 °C, dans un solvant miscible à l'eau ne pouvant pas être alkylé en présence d'une base.

12. Procédé selon la revendication 11 dans lequel ledit solvant est de l'acétonitrile et ladite base est de la triéthylamine.

13. Procédé selon la revendication 1 dans lequel ladite réaction de déprotection des groupes hydroxyles en positions 3 et 17 lors de l'étape c) ou lors de l'étape e), est effectuée à une température comprise entre 5 et 40 °C, par traitement du produit de formule (IV) ou de formule (VI) respectivement, avec des solutions aqueuses d'un sel d'acide ou d'un acide protique, ayant un pH inférieur ou égal à 4.

14. Procédé selon la revendication 13 dans lequel ladite réaction est effectuée dans un environnement de solution aqueuse biphasique acide/chlorure de méthylène.

15. Procédé selon la revendication 1 dans lequel les réactions des étapes a), b) et c) et la réaction de décarbonylation de l'étape e) sont effectuées dans une atmosphère d'argon ou d'azote inerte.

16. Procédé selon la revendication 1 dans lequel les réactions des étapes a), b) et c) et la réaction de décarbonylation de l'étape e) sont effectuées dans des conditions permettant de minimiser la présence d'eau dans l'environnement de la réaction.

17. Produits de formule générale (IV), (V) et (VI) en tant qu'intermédiaires dans le procédé tel que défini dans les revendications 1 à 16.
